(19) Europäisches Patentamt

European Patent Office

Office européen des brevets

(11) **EP 1 615 069 A1**

(12) **DEMANDE DE BREVET EUROPEEN**

(43) Date de publication:
**11.01.2006 Bulletin 2006/02**

(51) Int Cl.:
*G02C 11/04* *(2006.01)*

(21) Numéro de dépôt: **05014320.5**

(22) Date de dépôt: **01.07.2005**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **06.07.2004 CA 2472413**

(71) Demandeur: **Lavoie, David**
**Laval QC H75 1J8 (CA)**

(72) Inventeur: **Lavoie, David**
**Laval QC H75 1J8 (CA)**

(74) Mandataire: **de Beaumont, Michel**
**Cabinet Michel de Beaumont**
**1, rue Champollion**
**38000 Grenoble (FR)**

(54) **Lunettes de ski avec dispositif d'éclairage**

(57) Les lunettes de ski ont toujours servi à protéger les yeux des skieurs, à améliorer la vue de ceux-ci et à ajouter un élément d'esthétisme à un habit de ski. Les lunettes de ski sont utilisées très souvent durant la saison d'hiver. Comme on le sait, les journées d'hiver sont habituellement plus courtes, donc il fait noir beaucoup plus tôt. C'est pourquoi dans la présente invention, les lunettes de ski sont munies de lumières (D.E.L.) (diode émettant de la lumière) qui optimisent énormément la vision du skieur. Elles ne sont plus passives, elles deviennent actives et modifient l'environnement de leur détenteur. Elles éclairent tous les obstacles qui se trouvent devant lui. Non seulement il voit mieux les autres skieurs qui l'entourent, mais eux se rendent compte de sa présence beaucoup plus vite et cela a pour effet d'éviter des collisions et des chutes douloureuses. De plus, avec la multitude de lumières de couleurs disponibles, le nombre et la façon de les placer sur les lunettes; cela accentue les différences entre les habits des skieurs. Même à un kilomètre, il vous est possible de distinguer votre ami des autres skieurs. Le concept de lunettes en question ici est constitué d'un fil qui a pour fonction d'acheminer l'électricité de la pile vers les lunettes. Ce fil peut aussi servir de lien entre le manteau du skieur et les lunettes, donc elles ne se retrouvent plus jamais hors de la portée du skieur.

Fig. 1

EP 1 615 069 A1

## Description

La présente invention se rapporte à un accessoire de sport servant à la protection des yeux ainsi qu'à l'éclairement du paysage qui entoure le sportif qui possède les lunettes décrites ici.

Les lunettes de ski ont toujours servi à protéger les yeux des skieurs, à améliorer la vue de ceux-ci et, de par leur beauté, à ajouter un élément d'esthétisme à un habit de ski. Elles protègent les yeux de la neige, du froid, des branches d'arbre et des différents projectiles pouvant blesser les yeux. Elles améliorent la vue avec leurs différentes sortes de lentilles qui, de par leurs couleurs ou leurs polarités, réussissent à optimiser les paysages qui entourent les skieurs. Leurs formes, leurs couleurs et leurs attributs donnent à leur propriétaire un élément à la mode qui se trouve à l'endroit le plus en vue sur ceux-ci, le centre de leur visage. Toutes ces qualités sont très louables, mais les lunettes ordinaires sont passives, elles ne modifient pas l'environnement de leur détenteur. De plus, elles n'ont habituellement aucun lien fiable avec le skieur. Ce qui fait qu'elles peuvent tomber et se retrouver loin de lui.

Les lunettes de ski sont utilisées très souvent durant la saison d'hiver. Comme on le sait, les journées d'hiver sont habituellement plus courtes, donc il fait noir beaucoup plus tôt. C'est pourquoi dans la présente invention, les lunettes de ski sont munies de lumières ( D.E.L. ) qui optimisent énormément la vision du skieur. Elles ne sont plus passives, elles deviennent actives et modifient l'environnement de leur détenteur. Elles éclairent tous les obstacles qui se trouvent devant lui. Non seulement il voit mieux les autres skieurs qui l'entourent, mais eux se rendent compte de sa présence beaucoup plus vite et cela a pour effet d'éviter des collisions et des chutes douloureuses. De plus, avec la multitude de lumières de couleurs disponibles, le nombre et la façon de les placer sur les lunettes; cela accentue les différences entre les habits des skieurs. Même à un kilomètre, il vous est possible de distinguer votre ami des autres skieurs. Ce qui ne serait pas possible en temps normal. Un avantage secondaire est même à considérer : il arrive fréquemment qu'en tombant sur la piste ou en retirant ses lunettes pour les nettoyer dans le remonte-pentes, que les lunettes du skieur tombent et se retrouvent loin de lui. Le skieur doit donc retourner les chercher. Une situation pour le moins désagréable. Le concept de lunettes en question ici est constitué d'un fil qui a pour fonction d'acheminer l'électricité de la pile vers les lunettes. Ce fil peut aussi servir de lien fiable entre le manteau du skieur et les lunettes, donc elles ne se retrouvent plus jamais hors de la portée du skieur.

Relativement aux dessins qui illustrent la réalisation de l'invention :

- la figure 1 représente une vue de l'arrière d'une réalisation
- la figure 2 représente une vue de l'arrière de ladite réalisation avec un ajout
- la figure 3 représente une vue de l'arrière de ladite réalisation ( photo )
- la figure 4 représente une vue de face de ladite réalisation ( photo )
- la figure 5 représente une vue du haut de ladite réalisation ( photo )
- la figure 6 représente une vue de face ( gros plan ) de ladite réalisation (photo)

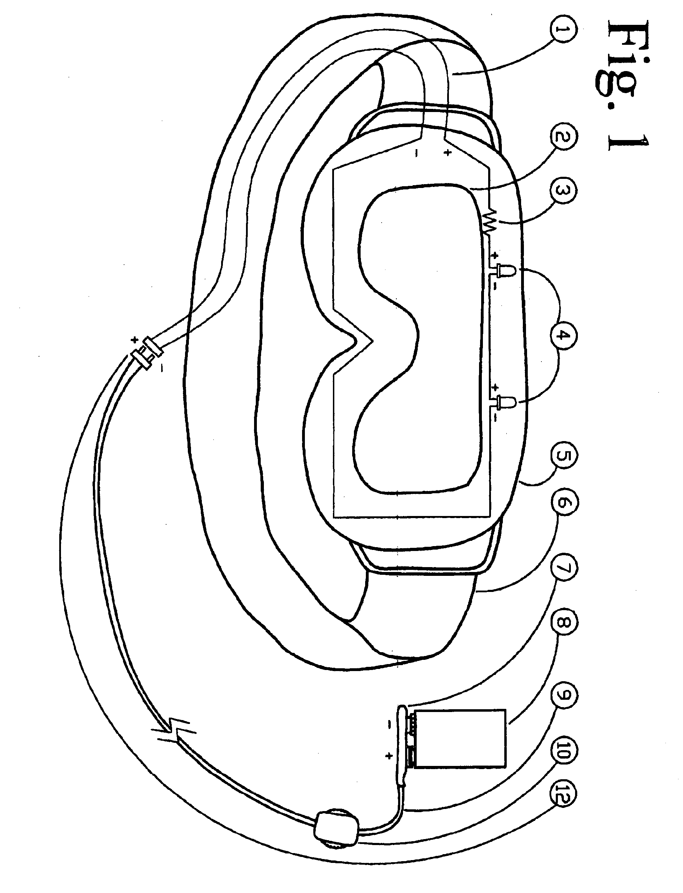

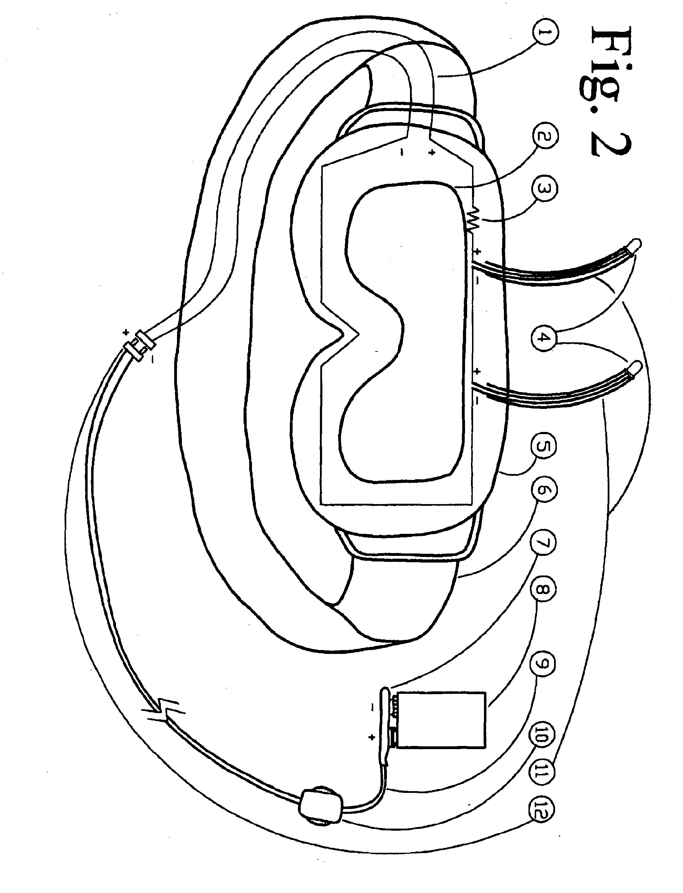

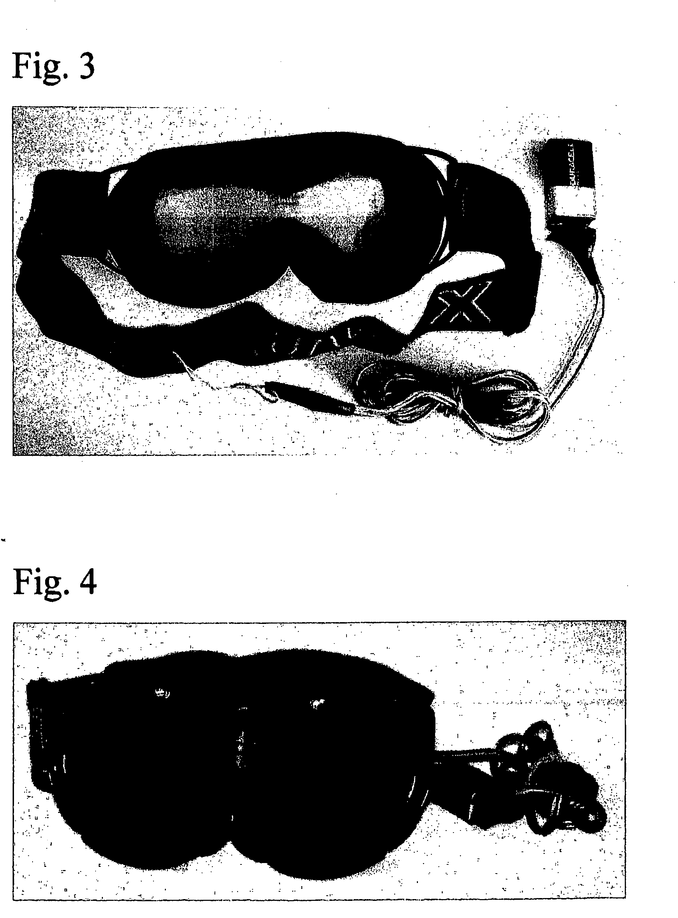

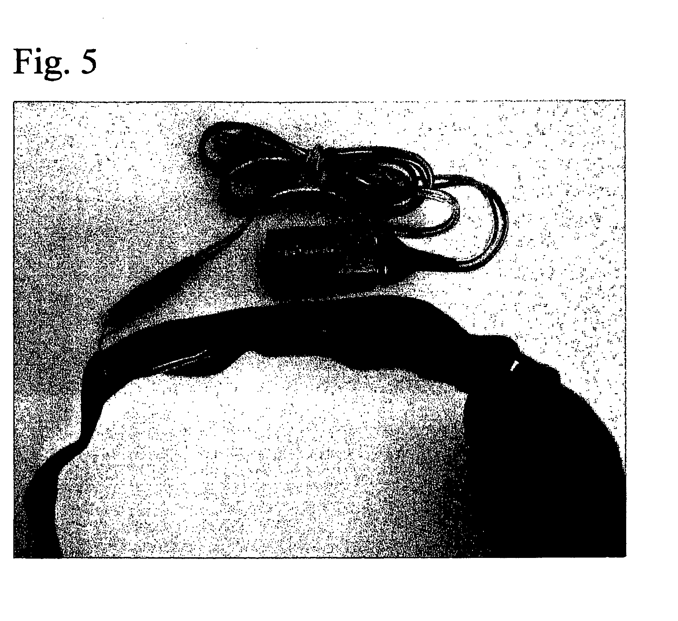

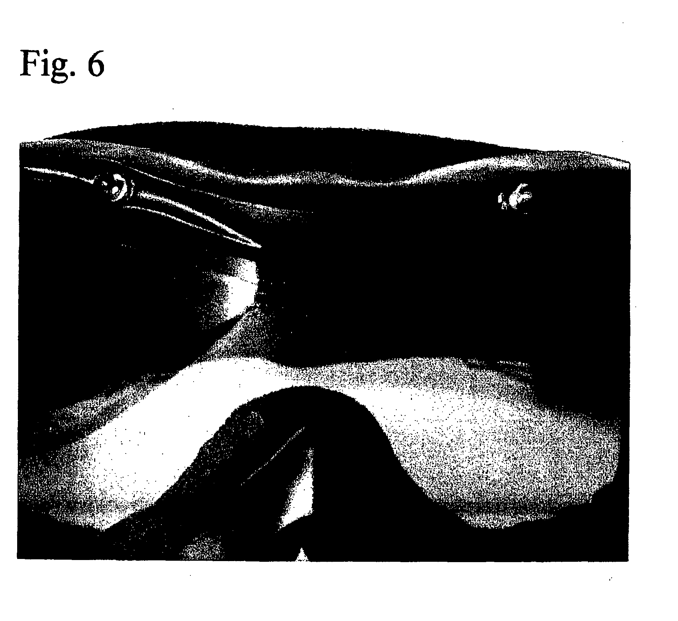

Les lunettes illustrées ( Fig. 1 à 6 ) comprennent une source d'électricité, dans ce cas-ci, une pile 9V #8. Il est préférable qu'elle soit rangée ailleurs que sur les lunettes pour éviter les blessures dues à un impact résultant d'une chute. De plus, le choix de la pile 9V a été fait en fonction de la duré d'utilisation. Il est aussi possible d'insérer des piles miniatures ( pour les montres ) dans la monture #5, mais c'est compliqué et inefficace par rapport à la duré. Celle-ci est branchée à un connecteur #7 pour pile 9V. Un fil double 14ga #9 multibrins ( généralement utilisé pour des haut-parleurs ) est soudé au connecteur #7. Quelques centimètres plus loin, un interrupteur à roulette #10 est raccordé au fil #9. Celui-ci sert à allumer et à éteindre les lumières. Avec sa roulette, il est facile à contrôler avec des gans d'hiver. De plus, la roulette ne tourne pas toute seule, contrairement à un bouton qui lui pourrait être actionné par inadvertance. Après l'interrupteur #10, le fil #9 continu sa route pour une distance de 137 cm ( 4,5 pieds ) vers la partie mâle d'un connecteur #12 en plastique à deux pines de fer. Ce connecteur est soudé au fil double #9 et a pour fonction de permettre à l'utilisateur de se départir du fil et de la pile s'il ne prévoit pas s'en servir. Les manteaux d'hiver sont souvent pourvue d'une poche interne qui se situe normalement à gauche de la poitrine. Si l'utilisateur range la pile dans cette poche, il n'y a pas de problème, mais s'il n'en n'a pas et qu'il doit la ranger dans sa poche de pantalon, la longueur du fil #9 devient importante. C'est pourquoi la longueur du fil a été déterminée à 4,5 pieds. Ceci accommode tout le monde, indépendamment de leur taille. De plus, une petite longueur supplémentaire a été rajouté pour éviter une tension indésirable ( étirement ) dans le fil. Ceci aurait pour effet de rendre inconfortable l'utilisateur et d'affaiblir le système électrique avec le temps. Après la partie mâle, il y a la partie femelle du connecteur #12. Celle-ci est branchée à la lunette parce qu'elle ne possède pas de pine de fer pouvant blesser l'utilisateur lors d'une chute, contrairement à la partie mâle. Un fil mono brin 22ga #1 est ensuite soudé à la borne positive de la partie femelle du connecteur #12. Ce fil passe par la bande élastique #6 et va vers le haut de l'intérieur de la monture #5. À cet endroit, le fil #1 est branché à une résistance #3. Pour savoir la valeur de la résistance qu'il faut brancher ici, voyez le tableau de valeurs un peu plus loin ainsi que la formule qui le suit. La première D.E.L. #4 est connectée par sa patte positive à la résistance #3. Ensuite, la patte négative est branchée à la patte positive de l'autre D.E.L. #4. Ce qui fait qu'elles sont en série. Si jamais d'autres D.E.L. devaient être disposées sur les lunettes, elles pourraient être ajoutées à cette série de la même façon que les deux suivantes sont connectées. Le fil **#1** sort de la patte négative de la deuxième D.E.L., fait le contour du bas de la monture **#5**, sans passer devant la lentille **#2,** et retourne vers la borne négative du connecteur **#12** en passant par la bande élastique **#6.** Le fil **#1** tient facilement sur la monture **#5** et sur la bande **#6** avec soit des gouttes de colle chaude, des petits crochets en plastique ou des trous d'enfilement. Les D.E.L. #4 sont placées dans des trous de leur taille dans la monture **#5**. De plus, il est préférable de leur donner un angle d'approximativement 10° vers l'extérieur verticalement et 15° vers l'extérieur horizontalement. Cela à pour but de ne pas aveugler quelqu'un qui regarde dans les yeux de l'utilisateur. Il y a une autre façon de placer les lumières #4, on peut les placer au bout de springs ou de tiges **#11** ( **Fig. 2 ).** Ces springs ont pour but d'imiter les antennes d'une abeille. Cela fait qu'au moment ou l'utilisateur bouge la tête, les springs et les D.E.L. bougent comme des antennes. Cette sorte de lunette vise un public plus jeune. Il est possible aussi de placer les D.E.L. de tel sorte qu'elles éclairent la monture **#5** elle-même. Cela a pour effet de faire comme si les lunettes étaient faites avec des néons. On peut même mettre un objet en plastique devant les D.E.L., ce qui fait que l'objet illumine. Par exemple un coeur, une tête de mort ou une insigne de compagnie. Cet objet en plastique peut même être rétractable. Il n'a qu'à être muni de pine de plastique pouvant entrer dans des trous fait à cet effet dans la monture **#5**. Cela rajoute à l'esthétisme. Le choix de D.E.L. augmente de beaucoup le temps d'utilisation avec une seule pile 9V. Par exemple, avec 4 D.E.L. ( total de 7,6V, 20mA et 152 mW ), il est possible de les utiliser pendant plus de 16 heures ( voir le graphique de décharge pour une pile 9V un peu plus loin ). Imaginez maintenant qu'à raison de quelques heures par journée de ski, il est possible d'utiliser 2 D.E.L. pendant toute une saison de ski, sans changer de pile. C'est pourquoi il vaut mieux utiliser des D.E.L. que des lumières ordinaires ( incandescentes ). Celles-ci ont besoin de plus de puissance, donc elles font que la pile dure moins longtemps. Elles gaspillent une trop grande partie de cette puissance en chaleur. Il faut aussi noter que tout ce système ne rajoute que 80 grammes ( 2,9 oz ) aux lunettes.

**TABLEAU DE DONNÉES SUR DIFFÉRENTES D.E.L.**

| Size | Color | Part No. | Chip | | Lens Color | VI(v) @20mA | | Iv(mcd) @20mA | | View angle 2θ1/2 | Normal Voltage (v) | Normal Current (mA) | Resistance needed (Ω) for 2 leds |
|------|-------|----------|------|------|------------|------|------|------|------|------|------|------|------|
| | | | Material | AP(nm) | | Min. | Max. | Min. | Typ. | | | | |
| 3mm | Red | LUE2043 | AlGaInp | 620 | Clear | 1.7 | 2.8 | 1100 | 1800 | 30 | 1,9 | 20 | 260 |
| | Yellow | LNY12243 | AlGaInP | 595 | Clear | 1.7 | 2.8 | 1100 | 2200 | 20 | 1,9 | 20 | 260 |
| | Green | LDGM2043 | InGan | 523 | Clear | 3.0 | 4.0 | 1500 | 2700 | 30 | 3,3 | 20 | 120 |
| | Blue | LDBK2043 | GaInN/GaN | 470 | Clear | 3.0 | 4.0 | 90 | 1100 | 30 | 3,3 | 20 | 120 |
| | Purple | LOUV2043 | InGan | 400 | Clear | 3.0 | 4.0 | 65 | 110 | 30 | 3,4 | 20 | 110 |
| | White | LWK2043 | GaInN/GaN | - | Clear | 3.0 | 4.0 | 160 | 2200 | 30 | 3,6 | 20 | 90 |
| 5mm | Red | LUR3333/S46 | GaAlAs | 660 | Clear | 1.5 | 2.4 | 900 | 1800 | 30 | 1,9 | 20 | 260 |
| | Yellow | LUY3333/S46 | AlGaInP | 595 | Clear | 1.7 | 2.8 | 1100 | 2200 | 30 | 1,9 | 20 | 260 |
| | Green | LUG3333/S46 | AlGaInP | 674 | Clear | 1.7 | 2.8 | 350 | 660 | 30 | 3,3 | 20 | 120 |
| | Blue | LSBK3333 | InGaN/SIC | 468 | Clear | 3.0 | 4.0 | 550 | 900 | 15 | 3,3 | 20 | 120 |
| | Purple | LDUV3333 | InGaN | 400 | Clear | 3.0 | 4.0 | 160 | 300 | 20 | 3,4 | 20 | 110 |
| | White | LWK3333-50 | InGaN/GaN | - | Clear | 3.5 | 4.0 | 650 | 900 | 60 | 3,6 | 20 | 90 |
| | Orange | LUE3333 | AlGaInP | 620 | Clear | 1.7 | 2.8 | 1800 | 3400 | 20 | 2 | 20 | 250 |
| 10mm | Red | LUR13633 | GaAlAs | 660 | Clear | 1.5 | 2.4 | 1600 | 3000 | 12 | 1,9 | 20 | 260 |
| | Yellow | LUY13633 | AlGaInP | 595 | Clear | 1.7 | 2.8 | 1500 | 3000 | 12 | 2,2 | 20 | 230 |
| | Green | LVG13633 | GaP | 565 | Clear | 1.7 | 2.8 | 400 | 700 | 12 | 2,2 | 20 | 230 |
| ( Les valeurs ci-haut sont une référence prise dans les données de la compagnie Ligitek ) | | | | | | | | | | | | | |

Formule mathématique pour savoir quelle résistance **#3** il faut mettre dans le circuit en série **(Fig. 1 et 2):**

Source = Voltage de la source ( V )
Nb del = Nombre de D.E.L. à installer
V del = Voltage d'une D.E.L. ( V )
A del = Ampérage d'une D.E.L. (mA)

$$( \text{Source} - ( \text{Nb del} \; X \; \text{V del} ) ) \; / \; ( \text{A del} \; / \; 1000 ) \; = \; (\Omega)$$
$$\text{Ex}: \quad (\quad 9V \; - \; (\quad 2 \quad X \; 1,9V \; )) \; / \; ( \; 20mA \; / \; 1000 ) \; = \; ( \; 260\Omega )$$

Note : Cette formule peut servir à trouver la résistance requise dans un circuit contenant d'autres sortes de lumières que des D.E.L.. Il suffit d'y insérer les bonnes valeurs.

TYPICAL DISCHARGE CHARACTERISTICS AT 21 °C (70°F)

**BREVETS ANTÉRIEURS**

**US 6390640 B1**
21. MAY 2002
LIGHTED MASK FOR UNDERWATER DIVERS
KEVIN WONG
**US 6554444 B2**
29 APR 2003
GAZING POINT ILLUNGNATING DEVICE
JUN-ICHI SHIMADA ,
**US 4254451**
3 MAR 1981

SEQUENTIAL FLASHING DEVICE FOR PERSONNAL ORNAMENTATION JAMES A. COCHRAN JR.

CES BREVETS ONT UNE RESSEMBLANCE AVEC LA PRÉSENTE DEMANDE , MAIS LES DOMAINES, LES UTILITÉS AINSI QUE LES REVENDICATIONS N'ONT RIEN À VOIR AVEC CELLES EN QUESTION ICI.

Les réalisations de l'invention, au sujet desquelles un droit exclusif de propriété ou de privilège est revendiqué, sont définies comme suit :

**Revendications**

1.  Des lunettes de ski avec dispositif d'éclairage composées :

    (Ces lunettes sont dites de ski, mais elles sont utilisées dans plusieurs autres sport : **le snowboard, la motoneige, le point-ball...** Elles comportent même parfois des pièces qui font qu'on l'appelle masque. )

    (a) des éléments habituellement utilisés dans une paire de lunettes de ce type : une monture **#5**, une lentille #2 et une bande élastique #6;
    (b) de une ou plusieurs D.E.L. **#4** ( diode émettant de la lumière ) placées à n'importe quel endroit sur la lunette;
    (c) d'une source d'électricité à pile **#8** branchée aux lumières **#4** avec un fil #9 muni d'un interrupteur **#10**, d'un connecteur #12 et d'une résistance **#3**.

2.  Les lunettes de ski avec dispositif d'éclairage, tel qu'elles sont définies dans la revendication 1, dont les sources de lumière #4 peuvent être de toutes sortes autres que des D.E.L..

3.  Les lunettes de ski avec dispositif d'éclairage, tel qu'elles sont définies dans la revendication 1, dont les sources de lumière **#4** peuvent être de toutes les couleurs, de toutes les formes clignotantes ou non.

4.  Les lunettes de ski avec dispositif d'éclairage, tel qu'elles sont définies dans la revendication 1, dont les sources de lumières **#4** peuvent être maintenues autour de ladite lunette par une ou plusieurs tige, tube ou spring #11.

5.  Les lunettes de ski avec dispositif d'éclairage, tel qu'elles sont définies dans la revendication 1, dont les sources de lumière **#4** peuvent être recouvertes d'objets décoratifs transparents rétractables ou non.

6.  Les lunettes de ski avec dispositif d'éclairage, tel qu'elles sont définies dans la revendication 1, dont la source d'électricité **#8** peut être de toutes intensités, de toute sorte et peut être faite pour se trouver plus ou moins près de ladite lunette.

Fig. 1

Fig. 2

## Fig. 3

## Fig. 4

Fig. 5

Fig. 6

**Office européen des brevets**

**RAPPORT DE RECHERCHE EUROPEENNE**

Numéro de la demande

EP 05 01 4320

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X,D | EP 1 134 491 A (KANSAI TECHNOLOGY LICENSING ORGANIZATION CO., LTD) 19 septembre 2001 (2001-09-19) | 1-3,5,6 | G02C11/04 |
| Y | * alinéas [0012], [0090], [0055] - [0058] * | 4 | |
| | ----- | | |
| X | US 5 353 378 A (HOFFMAN ET AL) 4 octobre 1994 (1994-10-04) | 1-3,5,6 | |
| Y | * colonne 2, ligne 18 - colonne 6, ligne 6; figures 1-3 * | 4 | |
| | ----- | | |
| X | US 4 822 161 A (JIMMY ET AL) 18 avril 1989 (1989-04-18) | 2,3,5,6 | |
| Y | * colonne 3, ligne 52 - colonne 4, ligne 50; figure 4 * | 4 | |
| | ----- | | |
| X | US 5 946 071 A (FELDMAN ET AL) 31 août 1999 (1999-08-31) * colonne 3 - colonne 4; figures 1-3 * | 2-6 | |
| | ----- | | |

**DOMAINES TECHNIQUES RECHERCHES (Int.Cl.7)**

G02C

Le présent rapport a été établi pour toutes les revendications

| Lieu de la recherche | Date d'achèvement de la recherche | Examinateur |
|---|---|---|
| Munich | 11 octobre 2005 | Jestl, M |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

    & : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 05 01 4320

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-10-2005

| Document brevet cité au rapport de recherche | | Date de publication | Membre(s) de la famille de brevet(s) | | Date de publication |
|---|---|---|---|---|---|
| EP 1134491 | A | 19-09-2001 | US | 2001021108 A1 | 13-09-2001 |
| US 5353378 | A | 04-10-1994 | WO | 9424663 A1 | 27-10-1994 |
| US 4822161 | A | 18-04-1989 | AUCUN | | |
| US 5946071 | A | 31-08-1999 | AUCUN | | |

EPO FORM P0460

Pour tout renseignement concernant cette annexe : voir Journal Officiel de l'Office européen des brevets, No.12/82